(19) Europäisches Patentamt
European Patent Office
Office européen des brevets

(11) **EP 4 185 389 B1**

(12) **FASCICULE DE BREVET EUROPEEN**

(45) Date de publication et mention
de la délivrance du brevet:
**21.08.2024 Bulletin 2024/34**

(21) Numéro de dépôt: **21755010.2**

(22) Date de dépôt: **16.07.2021**

(51) Classification Internationale des Brevets (IPC):
***A61Q 5/00*** *(2006.01)*     ***A61P 33/14*** *(2006.01)*
***A61K 8/44*** *(2006.01)*

(52) Classification Coopérative des Brevets (CPC):
**A61Q 5/00; A61K 8/44; A61K 8/442; A61P 33/14;**
A61K 2800/74

(86) Numéro de dépôt international:
**PCT/FR2021/051324**

(87) Numéro de publication internationale:
**WO 2022/018362 (27.01.2022 Gazette 2022/04)**

(54) **UTILISATION D'UN DERIVE DE GLYCINE BETAÏNE COMME AGENT PEDICULICIDE**

VERWENDUNG VON GLYCINE BETAIN-DERIVATEN ALS PÄDIKULISCHE MITTEL

USE OF A GLYCINE BETAINE DERIVATIVE AS PEDICULICIDAL AGENT

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **22.07.2020 FR 2007689**

(43) Date de publication de la demande:
**31.05.2023 Bulletin 2023/22**

(73) Titulaire: **Surfactgreen
60201 Compiegne Cedex (FR)**

(72) Inventeurs:
• **GALLE, Francis
35708 Rennes Cedex 7 (FR)**
• **ROUSSEL, Xavier
72000 LE MANS (FR)**

• **PESSEL, Freddy
35708 Rennes Cedex 7 (FR)**
• **BOYERE, Cédric
35000 RENNES (FR)**

(74) Mandataire: **Renard, Emmanuelle
Renard IP
4 Bis Avenue de Lorraine
92380 Garches (FR)**

(56) Documents cités:
EP-A1- 3 584 303     WO-A1-2005/121294
FR-A1- 2 949 182     FR-A1- 2 996 453
FR-A1- 3 088 930     US-A1- 2002 025 336
US-A1- 2010 273 694     US-A1- 2017 087 074
US-B1- 6 346 259

**Description**

OBJET DE L'INVENTION

L'invention a pour objet une composition tensioactive renfermant au moins un sel d'ester ou d'amide de glycine bétaïne, ou une composition topique la contenant, pour son utilisation comme agent pédiculicide. Elle a également pour objet un procédé pour prévenir et/ou traiter les infestations par les poux de tête et/ou les lentes, comprenant l'application sur une surface à traiter, en particulier sur les cheveux et le cuir chevelu, d'une composition topique renfermant une composition tensioactive telle que définie ci-dessus.

ARRIERE-PLAN DE L'INVENTION

Les poux de tête constituent un fléau qui reste difficile à éradiquer malgré la diversité des traitements disponibles sur le marché. Dans les deux lieux habituels de transmission que sont la collectivité d'enfants et la famille, la contamination est avant tout inter humaine et se fait directement par contact, même bref, d'un sujet parasité à un autre sujet, quelle que soit son origine socio-économique. Une fois contaminé, le sujet est rapidement infesté, puisque la femelle pond 4 à 10 oeufs ou lentes par jour pendant 3 à 4 semaines, chaque lente formant au bout de 7 à 9 jours une larve qui devient adulte environ une semaine plus tard. Contrairement au pou de corps (*Pediculus humanus var corporis*), le pou de tête (*Pediculus humanus var capitis*) ne transmet pas d'agents pathogènes. Il entraîne néanmoins des démangeaisons, essentiellement dans la nuque et autour des oreilles, dues à la présence de substances allergènes dans la salive des parasites.

Parmi les méthodes utilisées pour lutter contre les poux, on recense :

- les traitements chimiques à base d'un pédiculicide agissant sur les poux et éventuellement aussi sur les lentes, tel que les composés organophosphorés, dont le malathion, les composés organochlorés, tels que le lindane, ainsi que les dérivés du pyrèthre et les pyréthrinoïdes de synthèse, dont la perméthrine, la d-phénothrine et la dépalléthrine qui sont éventuellement utilisés en association avec le butoxyde de pipéronyle.
- les traitements mécaniques, au moyen de peignes spécifiques,
- les moyens physiques, à l'aide de compositions de corps gras, tels que des huiles végétales ou de silicone, du squalane ou encore des esters d'acides gras, qui obstruent les voies respiratoires des poux, aboutissant à leur mort.

Ces méthodes présentent toutefois plusieurs inconvénients. En effet, les traitements chimiques à base d'organophosphorés ou d'organochlorés peuvent poser des problèmes de toxicité qui empêchent notamment leur utilisation par des femmes enceintes ou des enfants. En outre, l'efficacité des pédiculicides a diminué depuis quelques années du fait que les poux ont commencé à développer une résistance vis-à-vis de ces composés. Enfin, leur impact environnemental est critiqué.

De leur côté, les traitements physiques et mécaniques ne présentent pas d'effet indésirable sur la santé ni d'apparition de résistances, mais ils ne sont souvent pas suffisamment efficaces, notamment sur les lentes, et nécessitent de répéter leur application à intervalles réguliers. Surtout, l'efficacité des traitements physiques nécessite un temps de pose d'au moins huit heures. Pour toutes ces raisons, il en résulte une compliance souvent insuffisante du traitement, conduisant à une réinfestation. Par ailleurs, les huiles utilisées dans cette application ont tendance à rendre les cheveux gras et rendent ces produits difficiles à rincer, ce qui détourne certains consommateurs de leur utilisation.

Dans ce contexte, il reste nécessaire de disposer d'une nouvelle composition pédiculicide qui ne présente pas de problèmes de toxicité pour l'homme et l'environnement et qui permette d'exterminer les poux de manière efficace, typiquement en une seule application et après un temps de pose réduit. Les documents FR-A-2 996 453 , US-A-2002 025336 et FR-A-2 949 182 décrivent des compositions tensioactives pour traiter les poux. Néanmoins aucun ne décrit de dérivés de glycine bétaïne.

La Demanderesse a maintenant découvert, de façon inattendue et surprenante, que certains tensioactifs cationiques biodégradables à base de dérivés de glycine bétaïne présentaient des propriétés pédiculicides intéressantes. Il a en particulier été démontré que ces composés permettaient d'éradiquer au moins 90% voire 100% des poux, en quelques minutes, typiquement en moins de 15 minutes, voire en 10 minutes. Ces composés présentent par ailleurs une solubilité dans l'eau permettant de les formuler aisément et de les rincer facilement.

Il a déjà été suggéré que des tensioactifs pouvaient présenter des propriétés pédiculicides. Le brevet FR 2 996 453 divulgue ainsi l'utilisation comme pédiculicide d'une combinaison de tensioactifs non ioniques et anioniques. L'efficacité de ce mélange reste toutefois modérée, puisque seulement 55% des poux sont éliminés après 30 minutes, sauf à lui adjoindre des particules colloïdales de silice. Par ailleurs, il n'est pas démontré que des tensioactifs cationiques, a fortiori à base de dérivés de glycine bétaïne, pourraient présenter la même efficacité.

Des tensioactifs cationiques dérivés de glycine bétaïne, constitués de sels d'amides et d'esters de glycine bétaïne, ont par ailleurs été décrits pour leur utilisation dans des compositions insecticides (EP 3 584 303), éventuellement en combinaison avec des tensioactifs non ioniques du type alkyl polyglycosides (WO 2005/121294). Il n'est toutefois pas précisément suggéré de les utiliser dans des compositions destinées au traitement des poux, ni que ces tensioactifs puissent eux-mêmes présenter des propriétés pédiculicides.

RESUME DE L'INVENTION

L'invention a pour objet une composition tensioactive renfermant au moins un dérivé de glycine bétaïne de formule (1) : $X^{n-}[(CH_3)_3N^+-CH_2-COZ-R]_n$ où Z désigne un atome d'oxygène ou un groupe -NH, R est un groupe alkyle linéaire ou ramifié, saturé ou insaturé comprenant de 8 à 24 atomes de carbone, X est un anion organique ou inorganique, et n vaut 1 ou 2, ou une composition topique la contenant, pour son utilisation comme agent pédiculicide par application topique sur les cheveux et le cuir chevelu.

Elle a également pour objet un procédé pour prévenir et/ou traiter les infestations par les poux de tête et/ou les lentes, comprenant l'application sur une surface en tissu à traiter d'une composition topique renfermant une composition tensioactive telle que définie ci-dessus.

L'invention a encore pour objet l'utilisation de cette composition tensioactive, ou d'une composition topique la comprenant, pour éliminer les poux de tête et/ou les lentes sur une surface en tissu.

DESCRIPTION DETAILLEE

La présente invention porte sur une composition tensioactive renfermant au moins un dérivé de glycine bétaïne, qui est un sel d'ester ou d'amide de glycine bétaïne, pour son utilisation comme agent pédiculicide, c'est-à-dire comme médicament à application topique dans la prévention et/ou le traitement contre les poux de tête et/ou les lentes. Ces deux types de dérivés de glycine bétaïne, ainsi que leurs procédés de préparation seront maintenant décrits plus en détail.

Sels d'esters de glycine bétaïne

Les sels d'esters de glycine bétaïne peuvent être obtenus suivant un procédé comprenant les étapes successives consistant à :

(1) faire réagir de la glycine bétaïne ou l'un de ses sels avec au moins un alcool gras linéaire ou ramifié, saturé ou insaturé, renfermant de 8 à 24 atomes de carbone, en présence d'un acide organique ou inorganique ;
(2) refroidir le milieu réactionnel à une température de 20 à 90°C ; et
(3) récupérer la composition tensioactive ainsi obtenue.

La première étape de ce procédé consiste à estérifier la glycine bétaïne, ou triméthylglycine. La glycine bétaïne peut être d'origine végétale ou synthétique. Il est nécessaire de la protoner préalablement à l'aide d'un acide organique ou inorganique, dans la mesure où elle se présente sous forme zwitterionique (présence d'une fonction carboxylate). L'acide peut notamment être choisi parmi les acides inorganiques tels que l'acide chlorhydrique, l'acide sulfurique, les acides perhalohydriques, tel que l'acide perchlorique, et leurs mélanges. En variante, il peut être choisi parmi les acides organiques, tels que les acides alkyl sulfuriques, par exemple l'acide décyl ou lauryl sulfurique ; les acides arylsulfoniques, tels que l'acide benzène sulfonique, l'acide paratoluène sulfonique ; les acides alkylsulfoniques, tels que l'acide triflique, l'acide méthanesulfonique, l'acide éthanesulfonique, l'acide décylsulfonique, l'acide laurylsulfonique ou l'acide camphosulfonique ; l'acide sulfosuccinique ; et leurs mélanges. On peut également utiliser des acides de Lewis. De préférence, il s'agit d'un acide alkylsulfonique et en particulier de l'acide méthanesulfonique, dans la mesure où il est facilement biodégradable.

Au cours de l'estérification, la fonction acide de la bétaïne salifiée est mise à réagir avec un alcool gras, pour conduire à un ester de glycine bétaïne sous forme de sel. Par "alcool gras", on entend un alcool linéaire ou ramifié (de préférence linéaire), saturé ou insaturé, comprenant de 8 à 24 atomes de carbone. Des exemples de tels alcools gras peuvent être choisis dans le groupe constitué par : l'alcool caprylique (C8:0), l'octan-2-ol, l'alcool décylique (C10:0), l'alcool undécylique (C11:0), l'alcool laurique (C12:0), l'alcool myristique (C14:0), l'alcool cétylique (C16:0), l'alcool palmitoléique (C16:1), l'alcool stéarique (C18:0), l'alcool oléique (C18:1), l'alcool linoléique (C18:2), l'alcool linolénique (C18:3), l'alcool arachidique (C20:0), l'alcool arachidonique (C20:4), l'alcool béhénique (C22:0), le 2-hexyldécanol, le 2-octyldodécanol, le 2-décyltétradécanol et leurs mélanges. Des mélanges d'alcools gras utilisables peuvent être produits à partir d'une ou plusieurs huiles végétales et notamment d'huile de soja, d'olive, de tournesol, de maïs, de palme, de coprah, de coton, de lin, de germe de blé, de carthame ou de colza, par exemple.

La réaction d'estérification s'effectue généralement en l'absence de solvant. L'eau produite lors de la réaction contribue par ailleurs à la solubilisation de la glycine bétaïne dans le mélange réactionnel.

Pour la mise en oeuvre de cette réaction, on peut par exemple utiliser de 0,8 à 6,0 équivalents, de préférence de 0,8 à 2 équivalents, par exemple de 0,9 à 1,0 équivalent, ou en variante de 1,1 à 1,8 équivalent, préférentiellement dans ce cas de 1,2 à 1,6 équivalent et, mieux, de 1,3 à 1,5 équivalent d'alcool gras ou dans une seconde variante de 4,0 à 6,0 équivalents, préférentiellement dans ce cas de 4,5 à 5,5 équivalents et, mieux, de 4,8 à 5,2 équivalents d'alcool gras.

On utilise en outre avantageusement de 1,01 à 3,0 équivalents, de préférence de 1,5 à 2,0 équivalents, par exemple de 1,5 à 1,9 équivalent, et préférentiellement de 1,5 à 1,7 équivalent molaire d'acide organique ou inorganique, ou en variante de 1,02 à 1,08 équivalents, préférentiellement dans de cas de 1,03 à 1,07 équivalents et, mieux, de 1,04 à 1,06 équivalent molaire d'acide organique ou inorganique pour 1 équivalent de glycine bétaïne. L'estérification est réalisée à une température allant par exemple de 120 à 180°C, de préférence de 150 à 180°C. La réaction peut être réalisée sous pression atmosphérique ou de préférence sous pression réduite, par exemple à une pression de 10 à 600 mbar. La pression sera généralement d'autant plus faible que la longueur de chaîne de l'alcool gras mis en jeu est grande. Le milieu réactionnel est ensuite refroidi à une température de 20 à 90°C.

On récupère alors la composition tensioactive ainsi obtenue, qui renferme au moins un sel d'ester de glycine bétaïne de formule $X^{n-}[(CH_3)_3N^+-CH_2-COOR]_n$ où : X est un anion organique ou inorganique, R est un radical alkyle correspondant à l'alcool gras R-OH mis en oeuvre dans la réaction d'estérification, et n vaut 1 ou 2.

L'anion X est issu de l'acide mis en oeuvre dans la première étape du procédé et peut donc en particulier être un chlorure, un sulfate, un perchlorate, un ion alkylsulfate, notamment décylsulfate ou laurylsulfate, un ion arylsulfonate, notamment benzène sulfonate ou paratoluène sulfonate, un ion alkylsulfonate, notamment triflate, méthanesulfonate, éthanesulfonate, décylsulfonate, laurylsulfonate, camphosulfonate, ou un ion sulfosuccinate. On préfère selon l'invention que X soit choisi parmi les alkylsulfonates et les arylsulfonates, en particulier parmi les ions méthanesulfonate, éthanesulfonate, triflate, paratoluènesulfonate et camphosulfonate. Il s'agit avantageusement de l'ion méthanesulfonate.

Le radical R peut de son côté être choisi parmi les groupements octyle (C8:0), 1-méthylheptyle (C8r:0), décyle (C10:0), undécyle (C11:0), lauryle (C12:0), myristyle (C14:0), cétyle (C16:0), palmitoléyle (C16:1), stéaryle (C18:0), oléyle (C18:1), linoléyle (C18:2), linolényle (C18:3), arachidyle (C20:0), arachidonyle (C20:4), béhényle (C22:0), 2-hexyldécyle, 2-octyldodécyle et 2-décyltétradécyle.

Il est bien entendu que, dans le cas où plusieurs alcools gras sont mis en oeuvre dans la réaction d'estérification, la composition tensioactive obtenue selon l'invention comprendra plusieurs sels d'esters de glycine bétaïne. L'expression "un sel d'ester de glycine bétaïne" doit donc être comprise, dans le contexte de cette description et sauf indication contraire, comme se référant à un ou plusieurs de ces sels.

Le procédé décrit ci-dessus permet plus précisément d'obtenir une composition tensioactive renfermant, et généralement constituée par, les constituants suivants :

(a) au moins un sel d'ester de glycine bétaïne de formule (1) : $X^{n-}[(CH_3)_3N^+-CH_2-COO-R]_n$ où R est un groupe alkyle linéaire ou ramifié, saturé ou insaturé, comprenant de 8 à 24 atomes de carbone,
(b) au moins un alcool gras de formule R-OH,
(c) un acide organique ou inorganique de formule XH,
(d) un sel de glycine bétaïne de formule $X^{n-}[(CH_3)_3N^+-CH_2-COOH]_n$, et
(e) éventuellement, au moins un éther de dialkyle de formule R-O-R,

où X est un anion organique ou inorganique et n vaut 1 ou 2.

Cette composition tensioactive peut être utilisée telle quelle dans la présente invention. Dans ce cas, elle renferme généralement de 15 à 85 % en poids de sel d'ester de glycine bétaïne.

Dans une première variante, la composition tensioactive renferme :

(a) de 65 à 85% en poids, de préférence de 70 à 80% en poids, de sel d'ester de glycine bétaïne
(b) de 1 à 20% en poids, par exemple de 1 à 9% en poids ou de 10 à 20% en poids, d'alcool gras,
(c) de 1 à 20% en poids, par exemple de 5 à 15% en poids d'acide organique ou inorganique
(d) de 1 à 20% en poids, par exemple de 2 à 15% en poids, de sel de glycine bétaïne
(e) de 0 à 15 % en poids, par exemple de 2 à 10 % en poids, d'éther de dialkyle.

Dans une seconde variante, qui est préférée, la composition tensioactive renferme :

(a) de 15 à 50% en poids, de préférence de 20 à 30%, plus préférentiellement de 25 à 30% en poids, de sel d'ester de glycine bétaïne
(b) de 50 à 70% en poids, par exemple de 60 à 65% ou de 65 à 70% en poids, d'alcool gras,
(c) de 0 à 5% en poids, par exemple de 0 à 1% en poids d'acide organique ou inorganique
(d) de 0 à 3% en poids, par exemple de 0 à 1% en poids, de sel de glycine bétaïne

(e) de 0 à 15 % en poids, par exemple de 2 à 10 % en poids, d'éther de dialkyle.

Avantageusement, la composition tensioactive ne renferme pas d'autre constituant que les composants (a) à (e) ci-dessus, c'est-à-dire que le total des constituants (a) à (e) est égal à 100%. En variante, le procédé ci-dessus peut inclure une étape supplémentaire consistant à isoler le sel d'ester de glycine bétaïne présent dans cette composition, qui peut être utilisé en tant que tel dans la présente invention. Dans ce dernier cas, la composition tensioactive utilisée selon l'invention comprendra au moins 90%, de préférence au moins 95%, voire au moins 99% en poids de dérivé de glycine bétaïne.

Sels d'amides de glycine bétaïne

Ces dérivés de glycine bétaïne peuvent être préparés suivant un procédé comprenant les étapes successives consistant à :

(1) faire réagir de la glycine bétaïne ou l'un de ses sels avec un alcool linéaire ou ramifié, saturé ou insaturé, en $C_4$-$C_8$, en présence d'un acide organique ou inorganique, à une température allant par exemple de 100 à 180°C et sous pression réduite ;
(2) refroidir le milieu réactionnel à une température de 20 à 80°C ;
(3) ajouter une ou plusieurs alkylamines renfermant de 8 à 24 atomes de carbone ;
(4) éliminer l'alcool résiduel ; et
(5) récupérer la composition tensioactive ainsi obtenue.

La première étape de ce procédé consiste en une réaction d'estérification de la glycine bétaïne, qui peut être mise en oeuvre de manière similaire à l'obtention des esters de glycine bétaïne, excepté que l'on utilise un ou plusieurs alcool(s) linéaire(s) et/ou ramifié(s) en $C_4$-$C_8$ en présence de l'acide qui peut être choisi parmi ceux décrits précédemment. Des exemples de tels alcools comprennent le butanol, le pentanol, le 3-méthylbutan-1-ol (ou alcool isoamylique), l'alcool de fusel (mélange de pentanol, de 2-méthylbutan-1-ol et de 3-méthylbutan-1-ol), l'hexanol, l'heptanol, l'octanol et leurs mélanges. Par "butanol", on entend aussi bien dans cette description le *n*-butanol, l'isobutanol et le sec-butanol. Le butanol, et plus particulièrement le *n*-butanol, est préféré pour une utilisation dans cette invention. Cette réaction s'effectue généralement en l'absence de tout solvant, l'alcool utilisé constituant à la fois le réactif et le milieu. L'eau produite lors de la réaction contribue également à la solubilisation de la glycine bétaïne dans le mélange réactionnel. On peut généralement utiliser de 1,1 à 20 équivalents, par exemple de 2 à 4 équivalents, d'alcool linéaire ou ramifié en $C_4$-$C_8$ et de 1,0 à 1,5 équivalents d'acide sulfonique, par exemple de 1,0 à 1,2 équivalent et préférentiellement 1,1 équivalent d'acide sulfonique, pour 1 équivalent de glycine bétaïne. L'estérification peut être réalisée à une température de 100 à 180°C, préférentiellement de 100 à 160°C, plus préférentiellement de 120 à 150°C ou de 130 à 160°C sous pression atmosphérique ou sous pression réduite.
Le produit de la réaction d'estérification peut ou non être traité de manière à séparer le sel d'ester de glycine bétaïne formé du milieu réactionnel. Pour ce faire, on peut par exemple procéder à une filtration du milieu réactionnel, qui permet de séparer l'ester salifié précité, soluble dans l'alcool, des autres constituants qui ne sont pas solubles.
On ajoute ensuite soit au milieu réactionnel, soit à l'ester isolé, une ou plusieurs alkylamine(s) en C8-C24. Des exemples de telles amines sont : la dodécylamine ou laurylamine, tétradécylamine, l'hexadécylamine, l'octadécylamine, la docosanylamine, l'eicosanylamine et leurs mélanges.
Dans cette étape, l'alkylamine est avantageusement utilisée sous forme fondue. La quantité d'alkylamine(s) ajoutée peut par exemple représenter de 0,9 à 1,5 équivalent et de préférence de 1,0 à 1,2 équivalent pour 1 équivalent de glycine bétaïne initialement mise en oeuvre. Cette réaction d'aminolyse est typiquement réalisée à une température de 50 à 180°C et de préférence de 120 à 140°C, sous pression réduite, par exemple sous une pression de 1 à 30 mbar. En parallèle de la réaction d'aminolyse, l'alcool est éliminé par distillation sous pression réduite. La réaction d'aminolyse et la distillation se font pendant une durée de 1 à 7 heures, notamment de 3 à 5 heures.
On récupère alors la composition tensioactive ainsi obtenue.
Ce procédé permet d'obtenir une composition tensioactive comprenant, et de préférence constituée par :

(a) un ou plusieurs sel(s) d'amide de glycine bétaïne de formule (1) : $X^{n-}[(CH_3)_3N^+\text{-}CH_2\text{-}CONH\text{-}R]_n$ où R est un groupe alkyle linéaire ou ramifié, saturé ou insaturé, comprenant de 8 à 24 atomes de carbone ;
(b) un ou plusieurs sel(s) d'alkylammonium de formule (2) : $X^{n-}[NH_3^+R]_n$ où R est un groupe alkyle linéaire ou ramifié, saturé ou insaturé, comprenant de 8 à 24 atomes de carbone ;
(c) un ou plusieurs sel(s) d'ester de glycine bétaïne de formule (3) : $X^{n-}[(CH_3)_3 N^+\text{-}CH_2\text{-}COOR']_n$ où R' est un radical alkyle linéaire ou ramifié, saturé ou insaturé, contenant de 4 à 8 atomes de carbone ; et
(d) de la glycine bétaïne de formule (4) : $(CH_3)_3N^+\text{-}CH_2\text{-}COO^-$ ;

où X est un anion organique ou inorganique et n vaut 1 ou 2.

Cette composition tensioactive peut être utilisée telle quelle dans la présente invention. Dans ce cas, elle renferme généralement de 60 à 98% en poids, par exemple de 70 à 80% en poids, de sel d'amide de glycine bétaïne. Le constituant (b) peut représenter de 0 à 25% en poids, par exemple de 15 à 20% en poids, le constituant (c) de 0 à 15 % en poids, par exemple de 5 à 10% en poids, et le constituant (d) de 0 à 5% en poids, par rapport au poids total de la composition tensioactive. Avantageusement, celle-ci ne renferme pas d'autre constituant que les composants (a) à (d) ci-dessus. En variante, le procédé ci-dessus peut inclure une étape supplémentaire consistant à isoler le sel d'amide de glycine bétaïne présent dans cette composition, qui peut être utilisé en tant que tel dans la présente invention. Dans ce dernier cas, la composition tensioactive utilisée selon l'invention comprendra au moins 90%, de préférence au moins 95%, voire au moins 99% en poids de dérivé de glycine bétaïne.

Composition topique

La composition tensioactive décrite ci-dessus peut être utilisée elle-même en tant qu'agent pédiculicide, éventuellement après dilution dans l'eau pour atteindre un taux de matière sèche de 0,5 à 20% en poids, par exemple, de préférence de 1 à 10% en poids et, mieux, de 3 à 5% en poids. Dans une forme d'exécution préférée de l'invention, la composition tensioactive peut être mélangée à différents ingrédients pour former une composition désignée dans la présente description par "composition topique", dans la mesure où elle se présente sous la forme d'un produit adapté à une application topique sur la surface à traiter, telle que les cheveux et le cuir chevelu, en particulier sous la forme d'une lotion, d'un gel ou d'une crème. Il s'agit généralement d'une composition aqueuse ou d'une émulsion du type huile-dans-eau (H/E), eau-dans-huile (E/H) ou multiple (par exemple E/H/E). Dans le cas où la composition topique est formulée sous la forme d'une émulsion, on préfère qu'il s'agisse d'une émulsion huile-dans-eau. Dans tous les cas, la composition topique renferme généralement de 0,5 à 20% en poids, de préférence de 1 à 10% en poids et, mieux, de 3 à 5% en poids, de dérivé de glycine bétaïne.

Cette composition topique peut notamment être conditionnée dans un tube, un flacon, un flacon-pompe ou un pot. En variante, elle peut être conditionnée dans un récipient aérosol, afin d'assurer une application de la composition sous forme vaporisée. Dans ce dernier cas, la composition topique comprend de préférence au moins un agent propulseur.

La composition topique utilisée selon l'invention constitue généralement une composition cosmétique ou dermatologique et comprend dans ce cas un milieu cosmétiquement ou dermatologiquement acceptable, respectivement, c'est-à-dire un milieu compatible avec les cheveux et le cuir chevelu et ne générant pas d'irritation inacceptable du cuir chevelu ou d'autre effet indésirable après application sur les cheveux.

La composition topique renferme généralement une phase aqueuse comprenant de l'eau et éventuellement un ou plusieurs solvants hydrosolubles cosmétiquement acceptables choisis parmi les alcools en C1-C4, tels que l'éthanol, l'isopropanol, le tertio-butanol ou le n-butanol, les polyols tels que le glycérol, le propylèneglycol et les polyéthylèneglycols, et leurs mélanges. De préférence, la composition possède une teneur totale en eau comprise entre 5 et 95% en poids, de préférence entre 10 et 90%, par exemple entre 40 et 85% en poids, notamment entre 50 et 80% en poids par rapport au poids total de la composition. Le pH de cette composition varie généralement de 3 à 9, de préférence de 3 à 7, préférentiellement de 5 à 6.

Elle peut en outre comprendre au moins une phase grasse renfermant au moins un corps gras, de manière à former une émulsion. De préférence, le ou les corps gras sont choisis parmi les huiles. Par "huiles", on entend un composé liquide à température ambiante (25°C) et pression atmosphérique ($10^5$ Pa) qui, lorsqu'il est introduit à raison d'au moins 1% en poids dans l'eau à 25°C, n'est pas du tout soluble dans l'eau, ou soluble à hauteur de moins de 10% en poids, par rapport au poids d'huile introduit dans l'eau.

Comme exemples d'huiles, on peut notamment citer les alcools gras, les esters gras, les hydrocarbures d'origine végétale ou minérale, les triglycérides et les huiles végétales en contenant, et leurs mélanges. Comme alcools gras on peut notamment citer les alcools gras ramifiés et/ou insaturés en C10-C20 tels que l'octyldodécanol et l'alcool oléylique. Des exemples d'esters gras sont les esters d'acides et de mono-alcool choisis parmi : les mono- et polyesters d'acides linéaires saturés en C2-C10 (de préférence en C6-C10) et de mono-alcools linéaires saturés en C10-C18 (de préférence C10-C14), les mono- et polyesters d'acides linéaires saturés en C10-C20 et de mono-alcools ramifiés ou insaturés en C3-C20 (de préférence C3-C10) ; les mono- et polyesters d'acides ramifiés ou insaturés en C5-C20 et de mono-alcools ramifiés ou insaturés en C5-C20 ; les mono- et polyesters d'acides ramifiés ou insaturés en C5-C20 et de mono-alcools linéaires en C2-C4. Des exemples de tels esters gras sont notamment le mélange de caprate et caprylate de coco, le macadamiate d'éthyle, l'ester éthylique de beurre de karité, l'isostéarate d'isostéaryle, l'isononanoate d'isononyle, l'isononanoate d'éthylhexyle, le néopentanoate d'hexyle, le néopentanoate d'éthylhexyle, le néopentanoate d'isostéaryle, le néopentanoate d'isodécyle, le myristate d'isopropyle, le myristate d'octyldodécyle, le palmitate d'isopropyle, le palmitate d'éthylhexyle, le laurate d'hexyle, le laurate d'isoamyle, le nonanoate de cétostéaryle, le capylate de propylheptyle, l'adipate de disopropyle, l'adipate de diéthylhexyle, le sébaçate de diisopropyle et le sébaçate de diisoamyle.

Comme hydrocarbures, on peut citer le squalane (C30), notamment le squalane végétal extrait de l'huile d'olive, et l'hémisqualane (C15). Des exemples de triglycérides sont les triglycérides d'acides gras en C6-C12, tels que les triglycérides d'acides caprylique et caprique et la triheptanoïne. Des exemples d'huiles végétales sont notamment les huiles de germe de blé, de tournesol, d'argan, d'hibiscus, de coriandre, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de courge, de cassis, d'onagre, de lavande, de bourrache, de millet, d'orge, de quinoa, de seigle, de carthame, de bancoulier, de passiflore, de rosier muscat, d'Echium, de cameline ou de camélia.

Les corps gras peuvent représenter de 1 à 30% en poids, par rapport au poids total de la composition.

La composition topique utilisée selon l'invention peut également comprendre au moins un excipient usuel, notamment choisi parmi les tensioactifs non ioniques, les tensioactifs cationiques autres que les dérivés de glycine bétaïne, les tensioactifs anioniques, les tensioactifs amphotères ; les antioxydants ; les agents nacrants et/ou opacifiants ; les pigments ; les charges ; les agents séquestrants ; les épaississants ; les polymères non épaississants tels que les silicones aminées et/ou les polymères cationiques ; les parfums ; les conservateurs ; et leurs mélanges.

De préférence, les tensioactifs anioniques sont choisis parmi les sels d'acide alkylcarbonyliséthionique, tels que ceux identifiés sous les noms INCI SODIUM COCOYL ISETHIONATE et SODIUM COCOYL METHYL ISETHIONATE ; les sels d'acide lactylique, tels que le SODIUM LAUROYL LACTYLATE ; les sels d'acides aminés N-acylés, tels que le SODIUM LAUROYL GLYCINATE, le SODIUM LAUROYL SARCOSINATE, le SODIUM LAUROYL TAURATE et le SODIUM OLIVOYL GLUTAMATE ; les tensioactifs anioniques sulfatés, choisis notamment parmi les sels d'alkylsulfates, notamment le SODIUM COCO SULFATE et le POTASSIUM LAURYL SULFATE, les sels d'alkyl éther sulfates en C8-C14 tels que le SODIUM LAURYL ETHER SULFATE ; les savons sous forme de sels d'acides carboxyliques, notamment le SODIUM OLIVATE et le SODIUM PALMITATE ; et les tensioactifs alkyl éther carboxyliques, tels que les acides lauryl éther carboxyliques ou les lauryl éther carboxylates de sodium.

Le ou les tensioactifs non-ioniques utilisés dans la composition cosmétique sont préférentiellement choisis parmi : les alcools en C8 à C40, saturés ou non, linéaires ou ramifiés, éthérifiés par 1 à 100 moles d'oxyde d'éthylène, de préférence de 2 à 50, plus particulièrement de 2 à 40 moles d'oxyde d'éthylène, comportant de préférence une ou deux chaînes grasses ; les huiles végétales oxyéthylénées, saturées ou non, comprenant de 1 à 100, de préférence de 2 à 50 moles d'oxyde d'éthylène; les esters de sucrose tels que le sucrose stéarate et le sucrose distéarate, les alcools en C8 à C40, mono- ou polyglycérolés, comprenant de 1 à 50 moles de glycérol, de préférence de 1 à 10 moles de glycérol ; les amides d'acides gras en C8 à C30, saturés ou non, linéaires ou ramifiés, de poly(oxyde d'éthylène) ou de mono- ou diéthanol, notamment le cocamide MEA ou monoéthanolamide d'acides gras de coco; les esters d'acides en C8 à C30, saturés ou non, linéaires ou ramifiés, et de polyéthylène glycols ; les esters d'acides en C8 à C30, saturés ou non, linéaires ou ramifiés, et de sorbitol, de préférence oxyéthylénés ; et leurs mélanges.

Le ou les tensioactifs amphotères utilisés dans la composition cosmétique utilisée dans la présente invention peuvent être notamment des dérivés d'amines aliphatiques secondaire ou tertiaire, éventuellement quaternisées, dans lesquels le groupe aliphatique est une chaîne linéaire ou ramifiée comportant de 8 à 22 atomes de carbone, lesdits dérivés d'amines contenant au moins un groupe anionique tel que, par exemple, un groupe carboxylate, sulfonate, sulfate, phosphate ou phosphonate. On peut citer en particulier les alkyl(C8-C20)bétaïnes, les alkyl(C8-C20)sulfobétaïnes, les alkyl(C8-C20)amidoalkyl(C3-C8)bétaïnes, telles que la cocamidopropyl bétaïne, et les alkyl(C8-C20)-amidalkyl(C6-C8)sulfobétaïnes.

Le ou les tensioactifs cationiques éventuellement utilisés en plus des dérivés de glycine bétaïne peuvent être choisis parmi les sels d'amines grasses primaire, secondaire ou tertiaire, éventuellement polyoxyalkylénées, les sels d'ammonium quaternaire, et leurs mélanges.

Le ou les épaississants peuvent être choisis parmi les agents épaississants cellulosiques, par exemple l'hydroxyéthylcellulose, l'hydroxypropylcellulose et la carboxyméthylcellulose, la gomme de guar et ses dérivés, par exemple l'hydroxypropyl guar, les gommes d'origine microbienne, telle que la gomme de xanthane et la gomme de scléroglucane, les agents épaississants synthétiques tels que les homopolymères réticulés d'acide acrylique ou d'acide acrylamidopropanesulfonique, les polymères associatifs non ioniques, anioniques, cationiques ou amphotères. Parmi les polymères cationiques utilisables comme polymères épaississants, on peut citer plus particulièrement les polymères du type polyamine, polyaminoamide et polyammonium quaternaire, notamment les celluloses cationiques, les gommes de guar cationiques et les homopolymères ou copolymères d'halogénures de diméthyldiallyl-ammonium.

On préfère que la composition topique selon l'invention ne renferme pas d'actif pédiculicide neurotoxique. Par ce terme, on désigne une molécule qui, après avoir traversé la cuticule du pou, agit comme neurotoxique. Tel est notamment le cas des dérivés de pyrèthre et des pyréthrinoïdes, qui agissent en maintenant ouverts pendant un temps anormalement long les canaux sodiques voltage-dépendant, ce qui entraine la paralysie spastique et la mort du pou, ainsi que du malathion, qui agit comme inhibiteur de l'acétylcholinestérase, entraînant ainsi un blocage de l'influx nerveux et une accumulation du neuromédiateur qui conduit à une paralysie spastique mortelle. En revanche, la composition selon l'invention peut ou non renfermer un ou plusieurs composés capables de tuer les poux en les asphyxiant et/ou en les déshydratant par dissolution de leur exosquelette. Des exemples de tels composés sont les huiles de silicone, les huiles végétales, notamment l'huile de coco, et les esters d'acides gras.

La composition topique peut en variante ou en plus renfermer un ou plusieurs répulsifs choisis par exemple parmi les huiles essentielles contenant du linalol, telles que les huiles essentielles de lavande, de lavandin ou d'arbre à thé qui présentent une action répulsive sur les poux.

Procédé / Utilisation

L'invention a pour objet une composition tensioactive selon les revendications ou une composition dermatologique la contenant, pour son utilisation comme agent pediculicide par application topique sur les cheveux et le cuir chevelu. Cela permet d' éliminer les poux de tête et/ou les lentes.

L'invention porte également sur un procédé pour prévenir et/ou traiter les infestations par les poux de tête et/ou les lentes, comprenant l'application sur une surface en tissu à traiter d'une composition topique renfermant une composition tensioactive selon les revendications.

Elle porte également sur l'utilisation d'une composition tensioactive selon les revendications ou d'une composition topique la comprenant, pour éliminer les poux de tête et/ou les lentes sur une surface en tissu.

Il est également décrit ici l'utilisation de cette composition tensioactive pour la préparation d'une composition topique destinée à prévenir et/ou lutter contre les infestations par les poux de tête et/ou les lentes.

La composition topique utilisée selon l'invention peut être appliquée sur toute surface à traiter, notamment sur des surfaces en tissu, par exemple de sièges ou de literie, et/ou sur des surfaces corporelles, plus particulièrement sur les cheveux ou le cuir chevelu. Dans cette application particulière, la composition topique peut être appliquée sur cheveux secs ou mouillés, et de préférence sur cheveux secs. Un mode de réalisation consiste à appliquer sur les cheveux et le cuir chevelu une quantité efficace de la composition topique, par pulvérisation ou application manuelle, à malaxer les cheveux et/ou répartir la composition sur la chevelure à l'aide d'un peigne, à laisser poser la composition sur les cheveux pendant une durée allant de 5 minutes à une heure, de préférence de 5 minutes à 30 minutes, plus préférentiellement de 5 minutes à 15 minutes, puis à rincer la chevelure à l'eau ou à l'aide d'un shampooing. Un peigne à poux peut être utilisé avant ou après rinçage de la composition afin d'éliminer les insectes morts et éventuellement leurs lentes.

Ce traitement peut être répété une à trois fois si nécessaire, avec un intervalle de temps allant de 1 jour à 1 mois entre chaque application.

EXEMPLES

L'invention sera mieux comprise à la lumière des exemples suivants, qui sont donnés à titre purement illustratif .

**Exemple 1 : Synthèse d'une composition tensioactive à base de sel d'ester de glycine bétaïne (GBE C12)**

De la glycine bétaïne (1,0 éq) et de l'alcool laurylique (0,9 éq) sont introduits dans un réacteur. La température de consigne au niveau du mélange est fixée à 170°C et la pression est réduite jusqu'à une valeur de 60 mbar. Une fois les consignes de pression et de température atteintes, une solution d'acide méthanesulfonique à 70% (1,6 éq) est ajoutée au mélange réactionnel. Dès que l'ajout est terminé, la température de consigne est ramenée à 150°C et la pression est maintenue à une valeur de 30 mbar. Cinq heures après le début de l'introduction de l'acide, on laisse refroidir le mélange réactionnel à 80°C, puis le produit est récupéré, refroidi jusqu'à température ambiante, et constitue la composition tensioactive selon l'invention, qui renferme les constituants suivants :

|  | Composition massique |
|---|---|
| Mésylate de bétaïnate de lauryle | 66,5% |
| Mésylate de glycine bétaïne | 17,1% |
| Alcool laurylique | 2,6% |
| Acide méthanesulfonique | 10,6% |
| Ether dilaurylique | 3,2% |

**Exemple 2 : Synthèse d'une composition tensioactive à base de sel d'amide de glycine bétaïne (GBA C12)**

De la glycine bétaïne (1,0 éq) et de l'hexanol (3,0 éq) sont introduits dans un réacteur surmonté d'un Dean-Stark rempli d'hexanol. Sur le couvercle du réacteur est fixée une ampoule de coulée isobare contenant une solution d'acide méthanesulfonique à 70 % (1,1 éq). Le mélange est agité et chauffé à 150°C sous pression réduite à 600 mbar. Une fois les conditions réactionnelles atteintes, la solution d'acide méthanesulfonique à 70 % est introduite progressivement dans le mélange réactionnel. Une fois l'ajout terminé, la pression est diminuée régulièrement jusqu'à atteindre 300 mbar afin d'accélérer l'élimination de l'eau et permettre le déplacement de l'équilibre vers l'ester de glycine bétaïne. Le taux de conversion est suivi par des analyses RMN [1]H.

La méthode par RMN consiste à réaliser un spectre $^1$H de l'échantillon dissous dans un mélange CDCl$_3$/CD$_3$OD (1/1, v /v) en prenant le signal du méthanol comme référence à 3,31 ppm. Les signaux caractéristiques des différents composés sont ensuite intégrés : MsOGBOC6 ( 4,35 ppm, s, 2 H), MsOGB (4,28 ppm, s, 2 H), hexanol (3,53 ppm, t, 2 H), méthanesulfonate (2,74 ppm, s, 3 H), éther dihexylique (3,40 ppm, t, 4 H), où XOGBOC6 désigne le sel de sulfonate de l'ester de glycine bétaïne formé et XOGB désigne le sulfonate de glycine bétaïne formé. Le signal caractéristique du méthanesulfonate tient compte à la fois de l'acide méthanesulfonique présent dans le milieu, mais également du méthanesulfonate qui est le contre-ion de la glycine bétaïne et du mésylate de bétaïnate d'hexyle (MsOGBOC6).

Le taux de conversion de la réaction est obtenu grâce aux valeurs d'intégration par le calcul suivant :

$$\eta = \frac{I_{MsOGBOC6}/2}{I_{MsOGB}/2 + I_{MsOGBOC6}/2} = \frac{I_{MsOGBOC6}}{I_{MsOGB} + I_{MsOGBOC6}}$$

où :

$\eta$ est le taux de conversion

$I_i$ est la valeur d'intégration du signal caractéristique du composé i.

Une fois que le taux de conversion de la réaction d'estérification atteint 96 %, le mélange réactionnel est laissé refroidir jusqu' à 60°C. Pendant cette phase de refroidissement, le montage Dean-Stark est remplacé par un montage de distillation et le réacteur est placé sous pression réduite afin d'éliminer une partie de l'hexanol et les traces d'eau restantes dans le mélange réactionnel. Une fois le mélange à 80°C, de la laurylamine (1,1 éq) préalablement fondue est ajoutée. Le mélange réactionnel est alors chauffé à 150°C sous pression réduite. La pression est progressivement diminuée jusqu'à 10 mbar. Après la distillation totale de l'hexanol (environ 4 heures), le mélange réactionnel est récupéré et constitue la composition tensioactive.

Cette dernière présente la composition massique suivante :

|  | Composition massique |
| --- | --- |
| Mésylate de bétaïnylaminododécane | 73,9% |
| Mésylate de laurylammonium | 14,2% |
| Mésylate de bétaïnate d'hexyle | 7,7% |
| Glycine bétaïne | 1,0% |
| Hexanol | 1,0% |
| Ether dihexylique | 2,2% |

**Exemple 3 : Test d'efficacité**

Les compositions tensioactives préparées comme décrit aux Exemples 1 et 2 ci-dessus ont été testées suivant les recommandations de l'ECHA relatives à l'évaluation de l'efficacité des produits biocides (Guidance on the Biocidal Products Régulation Volume II Efficacy - Assessment and Evaluation (Parts B+C), Version 3.0 Avril 2018).

Ces compositions ont ainsi été chacune pulvérisées, sous forme de solutions aqueuses à 3% en poids de matière sèche, dans 4 boîtes renfermant chacune 25 poux (*Pediculus humanus capitis*), puis on a évalué le pourcentage de mortalité après 5, 10 et 15 min. La moyenne du résultat obtenu dans les 4 boîtes a été calculée. Les résultats de ces essais sont présentés dans le Tableau 3 ci-dessous.

| Temps | Taux de mortalité (%) | |
|---|---|---|
| | GBE C12 | GBA C12 |
| 5 min | 94% | 77% |
| 10 min | 100% | 100% |
| 15 min | 100% | 100% |

Cet exemple démontre ainsi que les compositions tensioactives selon l'invention permettent d'éliminer la totalité des poux après seulement 10 minutes d'application.

### Exemple 4 : Exemple comparatif

Le test présenté à l'Exemple 3 a été reproduit en utilisant une même concentration de différents tensioactifs cationiques à la place des dérivés de glycine bétaïne selon l'invention.

Les résultats de ce test comparatif sont rassemblés dans le Tableau 4 ci-dessous :

| Temps | Taux de mortalité (%) | | |
|---|---|---|---|
| | Chlorure de benzalkonium | Chlorure de cétrimonium | Chlorure de dodécyltriméthylammonium |
| 5 min | 70 | 79 | 49 |
| 10 min | 80 | 94 | 78 |
| 15 min | 82 | 100 | 91 |

Les tensioactifs cationiques classiques s'avèrent ainsi moins efficaces que les composés selon l'invention. En outre, le temps nécessaire pour éliminer 100% des poux est de 60 min dans le cas du chlorure de benzalkonium et de 30 min dans le cas du chlorure de dodécyltriméthylammonium.

### Exemple 5 : Compositions anti-poux

Les produits ci-dessous peuvent être préparés à l'aide des compositions tensioactives selon l'invention, à base respectivement de sels d'ester ou d'amide de glycine bétaïne ou de leurs mélanges, identifiées ci-dessous par "Dérivé de glycine bétaïne". Les autres constituants de ces produits sont identifiés par leurs noms chimiques (en minuscules) ou leurs noms INCI (en majuscule).

| Shampooing anti-poux | |
|---|---|
| **Composants** | **% massique** |
| COCAMIDOPROPYL BETAINE | 20% |
| COCAMIDE MEA | 5% |
| Dérivé de glycine bétaïne | 3% |
| Chlorure de sodium | 1% |
| TOCOPHERYL ACETATE | 0,50% |
| COCO NUCIFERA OIL | 0,50% |
| BENZYL ALCOHOL | 0,60% |
| Potassium sorbate | 0,30% |
| Acide citrique | qsp pH 5,5 |
| Eau | qsp 100% |

| Shampooing anti-poux | |
|---|---|
| **Composants** | **% massique** |
| COCAMIDOPROPYL BETAINE | 24% |
| Dérivé de glycine bétaïne | 3% |
| TOCOPHERYL ACETATE | 0,50% |
| GLYCERYL OLEATE | 0,50% |
| BENZYL ALCOHOL | 0,60% |
| POTASSIUM SORBATE | 0,30% |
| TRISODIUM CITRATE | qsp pH 5,5 |
| Eau | qsp 100% |

| Lotion anti-poux | |
|---|---|
| **Composants** | **% massique** |
| Dérivé de glycine bétaïne | 3 |
| Glycérine | 3 |
| Propylène glycol | 2 |
| Eau | qsp 100% |

| Shampooing anti-poux | |
|---|---|
| **Composants** | **% massique** |
| COCAMIDOPROPYL BETAINE | 5 |
| Dérivé de glycine bétaïne | 3 |
| Glycérine | 3 |
| NaCl | 2,5 |
| Citrate de sodium | Qsp pH=5,5 |
| Propylène glycol | 2 |
| Eau | qsp 100% |

| Crème Anti-poux | |
|---|---|
| **Composants** | **% massique** |
| Huile de Tournesol | 10 |
| Alcool stéarylique | 6 |
| SPAN 60 | 3,9 |
| Dérivé de glycine bétaïne | 3 |
| Glycérine | 3 |
| Polysorbate 80 | 1,1 |
| Hydroxyethylcellulose | 0,5 |

(suite)

| Crème Anti-poux | |
| --- | --- |
| **Composants** | **% massique** |
| Acide citrique (4%) | Qsp pH=5,5 |
| Eau | Qsp 100% |

**Revendications**

1. Composition tensioactive renfermant au moins un dérivé de glycine bétaïne de formule (1) : $X^{n-}[(CH_3)_3N^+-CH_2-COZ-R]_n$ où Z désigne un atome d'oxygène ou un groupe -NH, R est un groupe alkyle linéaire ou ramifié, saturé ou insaturé comprenant de 8 à 24 atomes de carbone, de préférence de 10 à 18 atomes de carbone, X est un anion organique ou inorganique, et n vaut 1 ou 2, ou composition dermatologique la contenant, pour son utilisation comme agent pédiculicide par application topique sur les cheveux et le cuir chevelu.

2. Composition selon la revendication 1, **caractérisée en ce que** le radical R est choisi parmi les groupements octyle (C8:0), 1-méthylheptyle (C8r:0), décyle (C10:0), undécyle (C11:0), lauryle (C12:0), myristyle (C14:0), cétyle (C16:0), palmitoléyle (C16:1), stéaryle (C18:0), oléyle (C18:1), linoléyle (C18:2), linolényle (C18:3), arachidyle (C20:0), arachidonyle (C20:4), béhényle (C22:0), 2-hexyldécyle, 2-octyldodécyle et 2-décyltétradécyle.

3. Composition selon la revendication 1 ou 2, **caractérisée en ce que** l'anion X est choisi parmi un chlorure, un sulfate, un perchlorate, un ion alkylsulfate, notamment décylsulfate ou laurylsulfate, un ion arylsulfonate, notamment benzène sulfonate, paratoluène sulfonate, un ion alkylsulfonate, notamment triflate, méthanesulfonate, éthanesulfonate, décylsulfonate, laurylsulfonate, camphosulfonate, ou un ion sulfosuccinate, de préférence parmi les alkylsulfonates et les arylsulfonates, plus particulièrement parmi les ions méthanesulfonate, éthanesulfonate, triflate, paratoluènesulfonate et camphosulfonate, mieux, X est l'ion méthanesulfonate.

4. Composition selon l'une quelconque des revendications 1 à 3, **caractérisée en ce que** la composition tensioactive renferme, et de préférence est constituée par, les constituants suivants :

   (a) au moins un sel d'ester de glycine bétaïne de formule (1) : $X^{n-}[(CH_3)_3N^+-CH_2-COO-R]_n$ où R est un groupe alkyle linéaire ou ramifié, saturé ou insaturé, comprenant de 8 à 24 atomes de carbone,
   (b) au moins un alcool gras de formule R-OH où R est tel que défini ci-dessus,
   (c) un acide organique ou inorganique de formule XH, et
   (d) un sel de glycine bétaïne de formule $X^{n-}[(CH_3)_3N^+-CH_2-COOH]_{n'}$

   où X est un anion organique ou inorganique et n vaut 1 ou 2.

5. Composition selon la revendication 4, **caractérisée en ce que** la composition tensioactive renferme :

   (a) de 65 à 85% en poids, de préférence de 70 à 80% en poids, de sel d'ester de glycine bétaïne
   (b) de 1 à 20% en poids, par exemple de 1 à 9% en poids ou de 10 à 20% en poids, d'alcool gras,
   (c) de 1 à 20% en poids, par exemple de 5 à 15% en poids d'acide organique ou inorganique
   (d) de 1 à 20% en poids, par exemple de 2 à 15% en poids, de sel de glycine bétaïne
   (e) de 0 à 15 % en poids, par exemple de 2 à 10 % en poids, d'éther de dialkyle.

6. Composition selon la revendication 4, **caractérisée en ce que** la composition tensioactive renferme :

   (a) de 15 à 50% en poids, de préférence de 20 à 30%, plus préférentiellement de 25 à 30% en poids, de sel d'ester de glycine bétaïne
   (b) de 50 à 70% en poids, par exemple de 60 à 65% ou de 65 à 70% en poids, d'alcool gras,
   (c) de 0 à 5% en poids, par exemple de 0 à 1% en poids d'acide organique ou inorganique
   (d) de 0 à 3% en poids, par exemple de 0 à 1% en poids, de sel de glycine bétaïne
   (e) de 0 à 15 % en poids, par exemple de 2 à 10 % en poids, d'éther de dialkyle.

**7.** Composition selon l'une quelconque des revendications 1 à 3, **caractérisée en ce que** la composition tensioactive renferme, et de préférence est constituée par, les constituants suivants :

(a) un ou plusieurs sel(s) d'amide de glycine bétaïne de formule (1) : $X^{n-}[(CH_3)_3N^+\text{-}CH_2\text{-}CONH\text{-}R]_n$ où R est un groupe alkyle linéaire ou ramifié, saturé ou insaturé, comprenant de 8 à 24 atomes de carbone ;
(b) un ou plusieurs sel(s) d'alkylammonium de formule (2) : $X^{n-}[NH_3^+R]_n$ où R est un groupe alkyle linéaire ou ramifié, saturé ou insaturé, comprenant de 8 à 24 atomes de carbone ;
(c) un ou plusieurs sel(s) d'ester de glycine bétaïne de formule (3) : $X^{n-}[(CH_3)_3 N^+\text{-}CH_2\text{-}COOR']_n$ où R' est un radical alkyle linéaire ou ramifié, saturé ou insaturé, contenant de 4 à 8 atomes de carbone ; et
(d) de la glycine bétaïne de formule (4) : $(CH_3)_3N^+\text{-}CH_2\text{-}COO^-$ ;

où X est un anion organique ou inorganique et n vaut 1 ou 2.

**8.** Composition selon l'une quelconque des revendications 1 à 7, **caractérisée en ce que** la composition est appliquée sur les cheveux et le cuir chevelu en une quantité efficace, par pulvérisation ou application manuelle, les cheveux sont malaxés et/ou la composition est répartie sur la chevelure à l'aide d'un peigne, la composition est laissée sur les cheveux pendant une durée allant de 5 minutes à une heure, de préférence de 5 minutes à 30 minutes, plus préférentiellement de 5 minutes à 15 minutes, puis la chevelure est rincée à l'eau ou à l'aide d'un shampooing.

**9.** Procédé pour prévenir et/ou traiter les infestations par les poux de tête et/ou les lentes, comprenant l' application sur une surface en tissu à traiter d'une composition topique renfermant une composition tensioactive comprenant au moins un dérivé de glycine bétaïne de formule (1) : $X^{n-}[(CH_3)_3N^+\text{-}CH_2\text{-}COZ\text{-}R]_n$ où Z désigne un atome d'oxygène ou un groupe -NH, R est un groupe alkyle linéaire ou ramifié, saturé ou insaturé comprenant de 8 à 24 atomes de carbone, X est un anion organique ou inorganique, et n vaut 1 ou 2.

**10.** Utilisation d'une composition tensioactive renfermant au moins un dérivé de glycine bétaïne de formule (1) : $X^{n-}[(CH_3)_3N^+\text{-}CH_2\text{-}COZ\text{-}R]_n$ où Z désigne un atome d'oxygène ou un groupe - NH, R est un groupe alkyle linéaire ou ramifié, saturé ou insaturé comprenant de 8 à 24 atomes de carbone, X est un anion organique ou inorganique, et n vaut 1 ou 2, ou d'une composition topique la comprenant, pour éliminer les poux de tête et/ou les lentes sur une surface en tissu.

**Patentansprüche**

**1.** Tensidzusammensetzung, enthaltend mindestens ein Glycinbetainderivat der Formel (1): $[(CH_3)_3N^+\text{-}CH_2\text{-}COZ\text{-}R]_n X^{n-}$, wobei Z für ein Sauerstoffatom oder eine -NH-Gruppe steht, R eine lineare oder verzweigte, gesättigte oder ungesättigte Alkylgruppe mit 8 bis 24 Kohlenstoffatomen, vorzugsweise 10 bis 18 Kohlenstoffatomen, ist, X ein organisches oder anorganisches Anion ist und n den Wert 1 oder 2 hat, oder dermatologische Zusammensetzung, die sie enthält, zur Verwendung als pediculizides Mittel durch topische Anwendung auf die Haare und die Kopfhaut.

**2.** Zusammensetzung nach Anspruch 1, **dadurch gekennzeichnet, dass** der Rest R aus Octyl(C8:0)-, 1-Methylheptyl(C8r:0)-, Decyl(C10:0)-, Undecyl(C11:0)-, Lauryl(C12:0)-, Myristyl(C14:0)-, Cetyl(C16:0)-, Palmitoleyl(C16:1)-, Stearyl(C18:0)-, Oleyl(C18:1)-, Linoleyl(C18:2)-, Linolenyl(C18:3)-, Arachidyl(C20:0)-, Arachidonyl(C20:4)-, Behenyl(C22:0)-, 2-Hexyldecyl-, 2-Octyldodecyl- und 2-Decyltetradecylgruppen ausgewählt ist.

**3.** Zusammensetzung nach Anspruch 1 oder 2, **dadurch gekennzeichnet, dass** das Anion X aus einem Chlorid, einem Sulfat, einem Perchlorat, einem Alkylsulfat-Ion, insbesondere einem Decylsulfat- oder Laurylsulfat-Ion, einem Arylsulfonat-Ion, insbesondere einem Benzolsulfonat- oder para-Toluolsulfonat-Ion, einem Alkylsulfonat-Ion, insbesondere einem Triflat-, Methansulfonat-, Ethansulfonat-, Decylsulfonat-, Laurylsulfonat- oder Camphersulfat-Ion, oder einem Sulfosuccinat-Ion, vorzugsweise aus Alkylsulfonaten und Arylsulfonaten, spezieller aus Methansulfonat-, Ethansulfonat-, Triflat-, para-Toluolsulfonat- und Camphersulfat-Ionen, ausgewählt ist und noch besser X das Methansulfonat-Ion ist.

**4.** Zusammensetzung nach einem der Ansprüche 1 bis 3, **dadurch gekennzeichnet, dass** die Tensidzusammensetzung die folgenden Bestandteile enthält und vorzugsweise daraus besteht:

(a) mindestens ein Glycinbetainestersalz der Formel (1): $[(CH_3)_3N^+\text{-}CH_2\text{-}COO\text{-}R]_n X^{n-}$, wobei R eine lineare oder verzweigte, gesättigte oder ungesättigte Alkylgruppe mit 8 bis 24 Kohlenstoffatomen ist,

(b) mindestens einen Fettalkohol der Formel R-OH, wobei R wie oben definiert ist,
(c) eine organische oder anorganische Säure der Formel XH und
(d) ein Glycinbetainsalz der Formel $[(CH_3)_3N^+-CH_2-COOH]_nX^{n-}$,

wobei X für ein organisches oder anorganisches Anion steht und n den Wert 1 oder 2 hat.

5. Zusammensetzung nach Anspruch 4, **dadurch gekennzeichnet, dass** die Tensidzusammensetzung Folgendes enthält:

(a) 65 bis 85 Gew.-%, vorzugsweise 70 bis 80 Gew.-%, Glycinbetainestersalz,
(b) 1 bis 20 Gew.-%, beispielsweise 1 bis 9 Gew.-% oder 10 bis 20 Gew.-%, Fettalkohol,
(c) 1 bis 20 Gew.-%, beispielsweise 5 bis 15 Gew.-%, organische oder anorganische Säure,
(d) 1 bis 20 Gew.-%, beispielsweise 2 bis 15 Gew.-%, Glycinbetainsalz,
(e) 0 bis 15 Gew.-%, beispielsweise 2 bis 10 Gew.-%, Dialkylether.

6. Zusammensetzung nach Anspruch 4, **dadurch gekennzeichnet, dass** die Tensidzusammensetzung Folgendes enthält:

(a) 15 bis 50 Gew.-%, vorzugsweise 20 bis 30 Gew.-%, weiter bevorzugt 25 bis 30 Gew.-%, Glycinbetainestersalz,
(b) 50 bis 70 Gew.-%, beispielsweise 60 bis bis 65 oder 65 bis 70 Gew.-%, Fettalkohol,
(c) 0 bis 5 Gew.-%, beispielsweise 0 bis 1 Gew.-%, organische oder anorganische Säure,
(d) 0 bis 3 Gew.-%, beispielsweise 0 bis 1 Gew.-%, Glycinbetainsalz,
(e) 0 bis 15 Gew.-%, beispielsweise 2 bis 10 Gew.-%, Dialkylether.

7. Zusammensetzung nach einem der Ansprüche 1 bis 3, **dadurch gekennzeichnet, dass** die Tensidzusammensetzung die folgenden Bestandteile enthält und vorzugsweise daraus besteht:

(a) ein oder mehrere Glycinbetainamidsalz(e) der Formel (1): $[(CH_3)_3N^+-CH_2-CONH-R]_nX^{n-}$, wobei R eine lineare oder verzweigte, gesättigte oder ungesättigte Alkylgruppe mit 8 bis 24 Kohlenstoffatomen ist;
(b) ein oder mehrere Alkylammoniumsalz(e) der Formel (2): $[NH_3^+R]_nX^{n-}$, wobei R eine lineare oder verzweigte, gesättigte oder ungesättigte Alkylgruppe mit 8 bis 24 Kohlenstoffatomen ist;
(c) ein oder mehrere Glycinbetainestersalz(e) der Formel (3): $[(CH_3)_3N^+-CH_2-COOR']_nX^{n-}$, wobei R' ein linearer oder verzweigter, gesättigter oder ungesättigter Alkylrest mit 4 bis 8 Kohlenstoffatomen ist; und
(d) Glycinbetain der Formel (4): $(CH_3)_3N^+-CH_2-COO^-$;

wobei X für ein organisches oder anorganisches Anion steht und n den Wert 1 oder 2 hat.

8. Zusammensetzung nach einem der Ansprüche 1 bis 7, **dadurch gekennzeichnet, dass** die Zusammensetzung durch Sprühen oder manuelles Aufbringen in einer wirksamen Menge auf die Haare und die Kopfhaut aufgebracht wird, die Haare geknetet werden und/oder die Zusammensetzung mit einem Kamm über die Haare verteilt wird, die Zusammensetzung über einen Zeitraum im Bereich von 5 Minuten bis einer Stunde, vorzugsweise von 5 Minuten bis 30 Minuten, weiter bevorzugt von 5 Minuten bis 15 Minuten, auf den Haaren belassen wird und die Haare dann mit Wasser oder mit einem Shampoo gespült werden.

9. Verfahren zur Prävention und/oder Behandlung von Befall durch Kopfläuse und/oder Nissen, umfassend das Aufbringen einer topischen Zusammensetzung, die eine Tensidzusammensetzung enthält, die mindestens ein Glycinbetainderivat der Formel (1): $[(CH_3)_3N^+-CH_2-COZ-R]_nX^{n-}$ umfasst, wobei Z für ein Sauerstoffatom oder eine -NH-Gruppe steht, R eine lineare oder verzweigte, gesättigte oder ungesättigte Alkylgruppe mit 8 bis 24 Kohlenstoffatomen ist, X ein organisches oder anorganisches Anion ist und n den Wert 1 oder 2 hat, auf eine zu behandelnde Stoffoberfläche.

10. Verwendung einer Tensidzusammensetzung, enthaltend mindestens ein Glycinbetainderivat der Formel (1): $[(CH_3)_3N^+-CH_2-COZ-R]_nX^{n-}$, wobei Z für ein Sauerstoffatom oder eine -NH-Gruppe steht, R eine lineare oder verzweigte, gesättigte oder ungesättigte Alkylgruppe mit 8 bis 24 Kohlenstoffatomen ist, X ein organisches oder anorganisches Anion ist und n den Wert 1 oder 2 hat, oder einer topischen Zusammensetzung, die sie umfasst, zur Beseitigung von Kopfläusen und/oder Nissen auf einer Stoffoberfläche.

**Claims**

1. A surfactant composition including at least one glycine betaine derivative of formula (1): $[(CH_3)_3N^+-CH_2-COZ-R]_nX^{n-}$, where Z denotes an oxygen atom or an -NH group, R is a saturated or unsaturated, linear or branched, alkyl group comprising from 8 to 24 carbon atoms, preferably from 10 to 18 carbon atoms, X is an organic or inorganic anion and n has the value 1 or 2, or a dermatological composition containing it, for its use as pediculicidal agent by topical application to the hair and the scalp.

2. The composition as claimed in claim 1, **characterized in that** the radical R is chosen from the octyl (C8:0), 1-methylheptyl (C8r:0), decyl (C10:0), undecyl (C11:0), lauryl (C12:0), myristyl (C14:0), cetyl (C16:0), palmitoleyl (C16:1), stearyl (C18:0), oleyl (C18:1), linoleyl (C18:2), linolenyl (C18:3), arachidyl (C20:0), arachidonyl (C20:4), behenyl (C22:0), 2-hexyldecyl, 2-octyldodecyl and 2-decyltetradecyl groups.

3. The composition as claimed in claim 1 or 2, **characterized in that** the anion X is chosen from a chloride, a sulfate, a perchlorate, an alkyl sulfate ion, in particular a decyl sulfate or lauryl sulfate ion, an arylsulfonate ion, in particular a benzenesulfonate or para-toluenesulfonate ion, an alkylsulfonate ion, in particular a triflate, methanesulfonate, ethanesulfonate, decylsulfonate, laurylsulfonate or camphorsulfonate ion, or a sulfosuccinate ion, preferably from the alkylsulfonates and the arylsulfonates, more particularly from the methanesulfonate, ethanesulfonate, triflate, para-toluenesulfonate and camphorsulfonate ions; better still, X is the methanesulfonate ion.

4. The composition as claimed in any one of claims 1 to 3, **characterized in that** the surfactant composition includes, and preferably consists of, the following constituents:

    (a) at least one glycine betaine ester salt of formula (1): $[(CH_3)_3N^+-CH_2-COO-R]_nX^{n-}$, where R is a saturated or unsaturated, linear or branched, alkyl group comprising from 8 to 24 carbon atoms,
    (b) at least one fatty alcohol of formula R-OH where R is as defined above,
    (c) an organic or inorganic acid of formula HX, and
    (d) a glycine betaine salt of formula $[(CH_3)_3N^+-CH_2-COOH]_nX^{n-}$,

    where X is an organic or inorganic anion and n has the value 1 or 2.

5. The composition as claimed in claim 4, **characterized in that** the surfactant composition includes:

    (a) from 65% to 85% by weight, preferably from 70% to 80% by weight, of glycine betaine ester salt,
    (b) from 1% to 20% by weight, for example from 1% to 9% by weight or from 10% to 20% by weight, of fatty alcohol,
    (c) from 1% to 20% by weight, for example from 5% to 15% by weight, of organic or inorganic acid,
    (d) from 1% to 20% by weight, for example from 2% to 15% by weight, of glycine betaine salt,
    (e) from 0% to 15% by weight, for example from 2% to 10% by weight, of dialkyl ether.

6. The composition as claimed in claim 4, **characterized in that** the surfactant composition includes:

    (a) from 15% to 50% by weight, preferably from 20% to 30% by weight, more preferentially from 25% to 30% by weight, of glycine betaine ester salt,
    (b) from 50% to 70% by weight, for example from 60% to 65% or from 65% to 70% by weight, of fatty alcohol,
    (c) from 0% to 5% by weight, for example from 0% to 1% by weight, of organic or inorganic acid,
    (d) from 0% to 3% by weight, for example from 0% to 1% by weight, of glycine betaine salt,
    (e) from 0% to 15% by weight, for example from 2% to 10% by weight, of dialkyl ether.

7. The composition as claimed in any one of claims 1 to 3, **characterized in that** the surfactant composition includes, and preferably consists of, the following constituents:

    (a) one or more glycine betaine amide salt(s) of formula (1): $[(CH_3)_3N^+-CH_2-CONH-R]_nX^{n-}$, where R is a saturated or unsaturated, linear or branched, alkyl group comprising from 8 to 24 carbon atoms;
    (b) one or more alkylammonium salt(s) of formula (2): $[NH_3^+R]_nX^{n-}$, where R is a saturated or unsaturated, linear or branched, alkyl group comprising from 8 to 24 carbon atoms;
    (c) one or more glycine betaine ester salt(s) of formula (3): $[(CH_3)_3N^+-CH_2-COOR']_nX^{n-}$, where R' is a saturated or unsaturated, linear or branched, alkyl radical containing from 4 to 8 carbon atoms; and
    (d) glycine betaine of formula (4): $(CH_3)_3N^+-CH_2-COO^-$;

where X is an organic or inorganic anion and n has the value 1 or 2.

8. The composition as claimed in any one of claims 1 to 7, **characterized in that** the composition is applied to the hair and the scalp in an effective amount, by spraying or manual application, the hair is kneaded and/or the composition is distributed over the hair using a comb, the composition is left to stand on the hair for a period of time ranging from 5 minutes to one hour, preferably from 5 minutes to 30 minutes, more preferentially from 5 minutes to 15 minutes, and then the hair is rinsed with water or using a shampoo.

9. A method for preventing and/or treating infestations by head lice and/or nits, comprising the application, to a surface made of fabric to be treated, of a topical composition including a surfactant composition comprising at least one glycine betaine derivative of formula (1): $[(CH_3)_3N^+-CH_2-COZ-R]_nX^{n-}$, where Z denotes an oxygen atom or an -NH group, R is a saturated or unsaturated, linear or branched, alkyl group comprising from 8 to 24 carbon atoms, X is an organic or inorganic anion and n has the value 1 or 2.

10. The use of a surfactant composition including at least one glycine betaine derivative of formula (1): $[(CH_3)_3N^+-CH_2-COZ-R]_nX^{n-}$, where Z denotes an oxygen atom or an -NH group, R is a saturated or unsaturated, linear or branched, alkyl group comprising from 8 to 24 carbon atoms, X is an organic or inorganic anion and n has the value 1 or 2, or of a topical composition comprising it, for eliminating head lice and/or nits on a surface made of fabric.

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2996453 A
- US 2002025336 A
- FR 2949182 A
- FR 2996453
- EP 3584303 A
- WO 2005121294 A